# EUROPEAN PATENT APPLICATION

(11) **EP 1 769 782 A2**
(43) Date of publication of application: **04.04.2007**
(21) Application number: 06121609.9
(22) Date of filing: 02.10.2006
(51) Int. Cl.: A61F 17/00

(54) **Portable emergency kit**

(30) Priority: 03.10.2005 IT VR20050119
(71) Applicant: Pillonca, Marco, 37131 Verona (IT); Pillonca, Antonella, 09040 Villaputzu CA (IT)
(72) Inventor: Pillonca, Marco, 37131, Verona (IT)
(74) Representative: Fisauli, Beatrice A. M.

(57) **Abstract**

A portable emergency kit 1 contains, in a specially shaped casing, various components designed for medical first aid. In particular, upper special capsules 10 contain cotton wool, a lower container 4 contains a liquid disinfectant and there is a cavity 5 for positioning sticking plasters and/or bandages.

## Description

The object of this invention is a portable emergency kit that contains, in a specially shaped casing, various components designed for medical first aid.

It consists of a special stick, with relatively compact dimensions, that contains the basic instruments necessary for first aid in case of health emergencies such as scrapes, skin abrasions, etc.

As is well-known there are particular situations, such as sports exercises, special jobs performed outside the workshop and others, where one can easily suffer slight physical accidents such as abrasions or injuries of other types.

In these cases it is necessary to turn to medical care done using instruments such as cotton wool, disinfectants, sticking plaster and others, generally contained in first aid kits.

In cases where persons are outdoors or far away from their habitual worksites it is rare for them to carry these first aid kits with them these being, generally, rather bulky. This means that when first aid is necessary the persons are unable to lend it, either to themselves or to others, having to handle the situation with improper means.

Even when these persons carry the first aid kit with them this kit still remain bothersome and difficult to carry. When, for example, they perform amateur sports activities that call for moving substantial distances, such as riding mountain bikes, jogging, and other activities, it is unthinkable for them to carry first aid kits which quite often would, however, be rather useful.

Or in the case of hikes or holiday excursions, often with children, there are frequent occasions where persons may suffer abrasions or insect bites and may need disinfectants and cotton wool. Once again here too it is difficult to carry first aid kits and in the majority of circumstances the persons prefer to carry just a few objects with themselves, such as a disinfectant or cotton wool, without these, however, being in a specific container that functions to best preserve them.

The scope and function of this invention is to create a special container that contains the basic objects required for first aid and which is, at the same time, compact and easy to carry and thus can be comfortably carried by persons even in all those situations that require constant movement and moving away from areas where the instruments for first aid are easy to access.

These scopes and others are all achieved by an emergency kit, object of the invention, characterized by the fact that it contains a base body, with a basically cylindrical shape, including a container for liquids such as disinfectants or the like and having cavities for positioning first aid objects, including above said base body a cylindrical element for delivering the liquid and designed to be connected with a plug, basically cylindrical in form, including at its top a seat for positioning disks of cotton wool and, on the lower part, at least one circumferential seat, designed for inserting capsules of cotton wool.

It is possible, thanks to the particular structure and shape of the invention object of the patent, to always have on hand all the basic components necessary for medical first aid in case of slight injuries. In fact the emergency kit in question, thanks to its compact dimensions and particular exploitation of inner spaces, can be easily and comfortably carried no matter what activity the person is performing.

Other characteristics and details of the invention can be better understood from the following description, given as an example without being exclusive, that refers to the attached drawings where:
- fig. 1: gives a schematic axonometric exploded view of the kit, object of the invention, in a first embodiment;
- fig. 2: is a lateral schematic cross-section view of the kit in figure 1;
- fig. 3: is a cross-section view from above, through plane A-A, of the kit in figure 2;
- fig. 4: is a front view of the emergency kit according to a second embodiment.

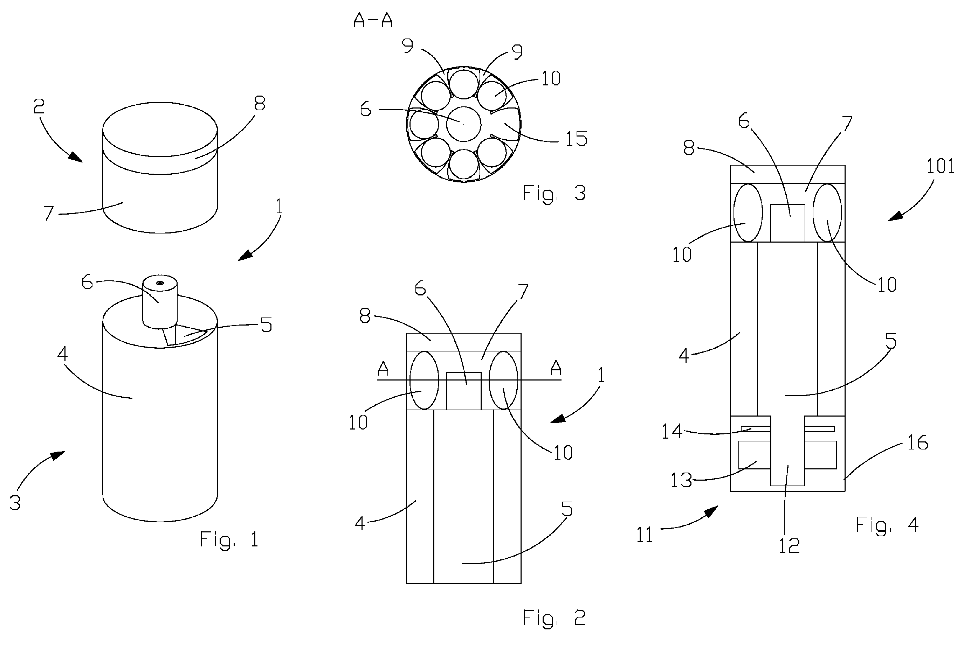

With reference to the attached figures No. 1 is used to indicate the emergency kit, object of the invention, as a whole, including base body 3, with basically cylindrical shape, and plug 2 with a particular shape.

Base body 3 contains, inside, space 4 that functions as a fluid container, in the specific case, for example, containing a disinfectant, that can exit from the upper face of cylindrical element 6 positioned on the upper base of said base body 3.

In addition said base body 3 has a cavity 5, with a basically angular cross-section shape, made from space 4 which acts as a container for sticking plaster and/or bandages or other components.

Plug 2, also with a basically cylindrical shape, is placed above this base body. Said plug 2 has, at its top, seat 8 designed to hold disks of cotton wool, easy to remove.

The lower part 7 of said plug 2 includes, as in figure 3, special clips 9 fastened to the circumferential wall of said lower part 7. Said clips 9 make it easy to insert and remove special capsules 10 which contain compressed and sterile cotton wool.

In a second embodiment 101 the previously described emergency kit 1 has, below base body 3, a special element 11 that includes insertion cylinder 12, designed to receive a particular disk 14 and a roll of band or sticking plaster 13 for injuries. Disk 14, in particular, has a blade on its outer surface that is specifically designed to cut the band from roll 13.

When it is not being used disk 14 and roll 13 are covered by special cover 16 which is fixed in a removable manner to base body 3.

Emergency kit 1, object of the invention, consequently permits persons to constantly have with themselves a handy and easy instrument for medical first aid with all the components necessary for dressing slight wounds such as scrapes, skin abrasions or other injuries.

A technician in the sector can also forecast several modifications or variants such as, for example, changing the shape from circular to polygonal, which are all to be held to be included within the scope of protection of the invention.

## Claims

1. Portable emergency kit (1) **characterized by** the fact that it contains base body (3) including container (4) for liquid disinfectants or the like and having cavities (5) for positioning first aid articles such as sticking plaster, bandages and the like; including, above said base body (3) a movable plug (2) with, at its top, seat (8) for positioning disks of cotton wool and, on the lower part (7) of said plug (2) at least one circumferential seat (15) designed for inserting at least one capsule of cotton wool (10).

2. Portable emergency kit (1) according to the preceding claim **characterized by** the fact that said base body (3), with basically cylindrical shape, includes at its top cylindrical element (6) for delivering the liquid.

3. Portable emergency kit (1) according to the preceding claims **characterized by** the fact that lower part (7) of said plug (2) includes at least one clip (9) fastened to the circumferential wall of said lower part (7) whereby this clip (9) permits insertion and removal of capsules (10) which contain compressed cotton wool.

4. Portable emergency kit (1) according to the preceding claims **characterized by** the fact that it has, below said base body (3), an element (11) that includes insertion cylinder (12), designed to receive a particular disk (14) and a roll (13) of band or sticking plaster for injuries.

5. Portable emergency kit (1) according to the preceding claims **characterized by** the fact that said disk (14) has a blade on the outer surface designed to cut the band from roll (13).

6. Portable emergency kit (1) according to the preceding claims **characterized by** the fact that when it is not being used disk (14) and roll (13) are covered by cover (16) fastened in a movable manner to base body (3).
